(19) Europäisches Patentamt
European Patent Office
Office européen des brevets

(11) Veröffentlichungsnummer: **0 303 243 A2**

(12) # EUROPÄISCHE PATENTANMELDUNG

(21) Anmeldenummer: 88112977.9

(51) Int. Cl.⁴: **A61K 37/02**

(22) Anmeldetag: 10.08.88

(30) Priorität: 14.08.87 DE 3727138

(43) Veröffentlichungstag der Anmeldung: 15.02.89 Patentblatt 89/07

(84) Benannte Vertragsstaaten: AT BE CH DE ES FR GB GR IT LI LU NL SE

(71) Anmelder: **HOECHST AKTIENGESELLSCHAFT**
**Postfach 80 03 20**
**D-6230 Frankfurt am Main 80(DE)**

(72) Erfinder: **Breipohl, Gerhard, Dr.**
**Geisenheimer Strasse 95**
**D-6000 Frankfurt am Main 71(DE)**
Erfinder: **Knolle, Jochen, Dr.**
**Höchster Strasse 21**
**D-6239 Kriftel(DE)**
Erfinder: **König, Wolfgang, Dr.**
**Eppsteiner Strasse 25**
**D-6238 Hofheim am Taunus(DE)**
Erfinder: **Linz, Wolfgang, Dr.**
**Gundelhardtstrasse 2**
**D-6233 Kelkheim (Taunus)(DE)**

(54) **Verwendung von atrionatriuretischem Faktor (ANF), dessen Teilsequenzen und Analogen zur Behandlung von Herzerkrankungen.**

(57) Die Erfindung betrifft die Verwendung von ANF, dessen Teilsequenzen und Analoga sowie deren physiologisch verträglichen Salze zur Prophylaxe bei ischämischen Herzzuständen und bei der Behandlung von Herzerkrankungen.

EP 0 303 243 A2

# Verwendung von atrionatriuretischem Faktor (ANF), dessen Teilsequenzen und Analogen zur Behandlung von Herzerkrankungen

Die Erfindung betrifft die Verwendung von ANF, dessen Teilsequenzen und Analoga sowie deren physiologisch verträgliche Salze zur Prophylaxe bei ischämischen Herzzuständen und bei der Behandlung von Herzerkrankungen.

Für diese neuartige Verwendung kommen beispielsweise Verbindungen der Formel I in Betracht,

$$\text{X-}\overset{3}{\text{V}}\text{-A-}A^1\text{-}A^2\text{-B-C-D-B-C-Gly-Ala-E-F-G-Leu-Gly-}\overset{19}{\text{Cys}}\text{-Z} \qquad (I)$$

in welcher

a) X Wasserstoff, ($C_1$-$C_5$)-Alkoxycarbonyl, ($C_6$-$C_{12}$)-Aryloxycarbonyl, ($C_7$-$C_{13}$)-Aralkyloxycarbonyl, ($C_1$-$C_6$)-Alkanoyl, ($C_7$-$C_{13}$)-Aroyl, Arginyl, Lysyl, $\epsilon$-Aminocaproyl, Arginyl-Arginyl, Arginyl-Lysyl, Lysyl-Arginyl oder Lysyl-Lysyl bedeutet, X fehlen kann, gegebenenfalls in $\alpha$-Stellung verzweigtes und gegebenenfalls in $\omega$-Stellung durch Amino oder Guanidino monosubstituiertes ($C_1$-$C_{12}$)-Alkylcarbonyl oder ($C_3$-$C_8$)-Cycloalkylcarbonyl bedeutet oder für Ser, Thr, Ser(Y), Thr(Y), Q oder Leu, jeweils in ihrer L-bzw. D-Konfiguration oder Ser-Ser, Thr-Thr, Ser-Thr, Q-Ser, Q-Thr, Thr-Q, Ser-Q, Ser(Y)-Ser oder Ser-Ser(Y) steht, wobei jede Aminosäure in ihrer L-bzw. D-Konfiguration vorliegen kann und die N-terminale Aminogruppe der Aminosäure oder des Dipeptid-Restes frei vorliegt (N-terminaler Rest = H) oder durch ($C_1$-$C_5$)-Alkoxycarbonyl, ($C_6$-$C_{12}$)-Aryloxycarbonyl, ($C_7$-$C_{13}$)-Aralkyloxycarbonyl, ($C_1$-$C_6$)-Alkanoyl, ($C_7$-$C_{13}$)-Aroyl, Arginyl, Lysyl, $\epsilon$-Aminocaproyl, Arginyl-Arginyl, Arginyl-Lysyl, Lysyl-Arginyl oder Lysyl-Lysyl acyliert ist;

V Cys oder, falls X fehlt, $\omega$-Thio($C_2$-$C_8$)alkylcarbonyl, wie beispielsweise Mercaptopropionsäure, Mercaptoessigsäure, Mercaptobuttersäure, bedeutet;

A Phe, Tyr(Me), Tyr($Bu^t$), 4-Halogenphenylalanin, Trp oder einen L-2-Thienylalanin-Rest bedeutet;

$A^1$ Gly, Ala oder D-Ala bedeutet;

$A^2$ Gly, Ala oder D-Ala bedeutet; oder

$A^1$ und $A^2$ zusammen gegebenenfalls verzweigtes $\omega$-Amino-($C_3$-$C_8$)-alkylcarbonyl bedeutet;

B Arg, Lys, Orn oder einen L-Homoarginin-Rest bedeutet;

C Ile, Met, Phe, Trp, Leu, Ser, Thr, Val, His, Pro, Asn, Ser($Bu^t$), Cyclohexylalanin-, tert. Butylglycin-, Neopentylglycin- oder einen L-2-Thienylalanin-Rest bedeutet;

D Asp, Glu, Gln, Asn, Phe, Leu, Ile, Trp, Pro, Tyr, Ala, Asp($OBu^t$), Asp(OBzl), Glu($OBu^t$), Glu(OBzl), einen 2-Thienylalanin-Rest, Aad, Tyr($Bu^t$) oder Tyr(Me) bedeutet, wobei die Aminosäuren jeweils in ihrer L- oder D-Konfiguration vorliegen können;

E Gln, Thr oder Pro bedeutet;

F Ser, Thr, Pro, Ala, Ser($Bu^t$) oder Thr($Bu^t$), jeweils in ihrer L- oder D-Konfiguration bedeutet;

G Gly, Ala oder D-Ala bedeutet;

Q einen Rest der Formel IV,

$$\begin{array}{ccccccc} & R^1 & & R^2 & & O & \\ & | & & | & & \| & \\ - & N & - & CH & - & C & - \end{array} \qquad (IV)$$

worin

$R^1$ und $R^2$ zusammen mit den diese Reste tragenden Atomen ein heterocyclisches mono-, bi- oder tricyclisches Ringsystem mit 3 bis 15 C-Atomen bilden,
bedeutet;

Y tert.-Butyl oder einen gegebenenfalls geschützten oder teilgeschützten Glycosylrest bedeutet;
und

Z für einen Rest der Formel II steht,

-H-I-J-K-L-M (II)

worin

H Asn, Ser, Q oder Thr, jeweils in ihrer L- oder D-Form, oder Gly bedeutet;

I Ser, Thr, Ala, Ser($Bu^t$), Thr($Bu^t$) oder Pro, jeweils in ihrer L- oder D-Form bedeuten;

J Phe, Trp, D-Phe, D-Trp, Tyr(Me), Cyclohexylalanyl oder einen 2-Thienylalanin-Rest bedeutet;

K Arg, Lys, Orn oder eine Bindung bedeutet;

L Q, Gly, Tyr, Tyr($Bu^t$), Tyr(Me) oder eine Bindung bedeutet;

M Arg-OH, Arg-$NH_2$, OH, OR, $NH_2$, NHR′, Gly-Lys-Arg-OH, Gly-Lys-Arg-$NH_2$ oder L-Argininol bedeutet;

Q wie oben definiert ist;

R unverzweigtes ($C_1$-$C_6$)-Alkyl bedeutet und

R′ -$[CH_2]_n$-$NH_2$ oder -$[CH_2]_n$-NH-C(NH)$NH_2$, wobei n ganzzahlig ist und für 3-8 steht, bedeutet;

sowie deren physiologisch verträgliche Salze, oder

b) X-$\overset{3}{V}$-A-$A^1$-$A^2$- und -Gly-Ala-E-F-G-Leu-Gly-$\overset{19}{Cys}$-Z fehlen und der Rest -B-C-D-B-C- für Verbindungen der Formel Ia

$R^N$-L′-N-$B^2$-$R^C$ (Ia)

steht, in welcher

$R^N$ für einen Rest der Formel IIa steht;

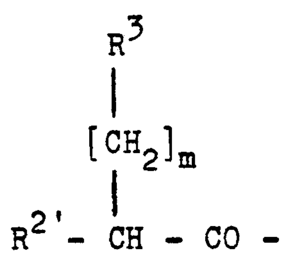

(IIa)

$R^{2'}$ für Wasserstoff oder einen Rest der Formel R″-$[A']_{n'}$-NH- steht;

$R^3$ Amino, Guanidino, ($C_1$-$C_3$)-Alkylamino oder Di-($C_1$-$C_3$)-alkylamino bedeutet;

m eine ganze Zahl von 1-6 bedeutet;

A′ für einen Rest der Formel -NH-$CR^4R^5$-CO- steht;

R″ Wasserstoff, ($C_1$-$C_6$)-Alkanoyl, ($C_7$-$C_{11}$)-Aroyl, das im aromatischen Teil gegebenenfalls durch ($C_1$-$C_4$)-Alkyl, ($C_1$-$C_4$)-Alkoxy, ($C_1$-$C_4$)-Alkylthio, Halogen, Carbamoyl, ($C_1$-$C_4$)-Alkoxycarbonyl und/oder Sulfamoyl mono- oder disubstituiert oder durch Methylendioxy monosubstituiert ist, ($C_5$-$C_7$)-Cycloalkyl-($C_1$-$C_3$)-alkanoyl oder ($C_6$-$C_{14}$)-Aryl-($C_1$-$C_3$)-alkanoyl bedeutet, wobei in den Resten R″ ≠ Wasserstoff eine -$CH_2$-Gruppe gegebenenfalls durch -O- oder -S- ersetzt ist;

n′ 0 oder 1 ist;

$R^4$ und $R^5$ gleich oder verschieden sind und Wasserstoff, ($C_1$-$C_6$)-Alkyl oder ($C_7$-$C_{11}$)-Aralkanoyl bedeuten;

L′ für den Rest einer lipophilen, neutralen α-Aminosäure, vorzugsweise für Pro, D-Pro oder einen Rest der Formel -NH-CH($R^6$)-CO- steht;

$R^6$ ($C_1$-$C_6$)-Alkyl, das gegebenenfalls durch Hydroxy, ($C_1$-$C_6$)-Alkoxy, ($C_1$-$C_6$)-Alkoxycarbonyl, Carbamoyl oder R″-NH monosubstituiert ist, wobei R″ wie oben definiert ist, aber nicht Wasserstoff sein kann, oder ($C_7$-$C_{11}$)-Aralkyl, das am Aromaten gegebenenfalls durch ($C_1$-$C_6$)-Alkoxy monosubstituiert ist, oder Indol-3-ylmethyl bedeutet;

N für den Rest einer neutralen α-Aminosäure, vorzugsweise für Pro, D-Pro oder einen Rest der Formel -NH-CH($R^7$)-CO- steht;

$R^7$ ($C_1$-$C_6$)-Alkyl, das gegebenenfalls durch Hydroxy, ($C_1$-$C_6$)-Alkoxy, ($C_1$-$C_6$)-Alkoxycarbonyl, Carbamoyl oder R″-NH monosubstituiert ist, wobei R″ wie oben definiert ist, aber nicht Wasserstoff sein kann, oder ($C_7$-$C_{11}$)-Aralkyl, das am Aromaten gegebenenfalls durch ($C_1$-$C_6$)-Alkoxy monosubstituiert ist, oder Indol-3-ylmethyl bedeutet;

$B^2$ für den Rest einer basischen α-Aminosäure, vorzugsweise für einen Rest der Formel IIIa

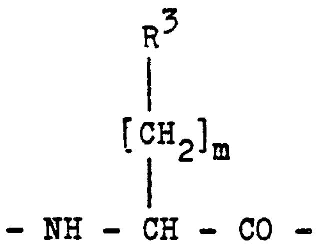

(IIIa)

worin $R^3$ und m wie oben definiert sind, steht;

$R^C$ für eine Rest der Formel $-NR^9-CH(R^8)-(CO)_p-R^1$ steht;

$R^8$ eine lipophile Seitenkette bedeutet, die vorzugsweise wie $R^7$ definiert ist, wobei vorhandene CH-, $CH_2$- oder $CH_3$-Reste in $\beta$-Stellung bezüglich -NH- gegebenenfalls monohydroxyliert sind, und

$R^9$ Wasserstoff bedeutet;

oder

$R^8$ und $R^9$ zusammen $-[CH_2]_3-$ oder $-[CH_2]_4-$ bedeuten;

p , 0 oder 1 ist;

$R^1$, im Falle von p = 0 für Wasserstoff, Hydroxy oder $(C_1-C_6)$-Alkoxy steht;

$R^1$ im Falle von p = 1 für $OR^{10}$ oder $NR^{10}R^{11}$ steht;

und

$R^{10}$ und $R^{11}$ gleich oder verschieden sind und Wasserstoff, $(C_1-C_6)$-Alkyl oder $(C_7-C_{11})$-Aralkyl bedeuten; oder $NR^{10}R^{11}$ für Pyrrolidino, Piperidino oder Morpholino steht;

oder

p = 1 ,ist,

$R''$ und $R^1$ gemeinsam eine Bindung bedeuten und die übrigen Reste wie oben definiert sind,

sowie deren physiologisch verträgliche Salze.

Als Salze kommen insbesondere Alkali- oder Erdalkalisalze, Salze mit physiologisch verträglichen Aminen und Salze mit anorganischen oder organischen Säuren wie z.B. HCl, HBr, $H_2SO_4$, $H_3PO_4$, Maleinsäure, Fumarsäure, Citronensäure, Weinsäure, Essigsäure in Frage. Die Chiralitätszentren in den neuen Peptiden können jeweils R-, S- oder R,S-Konfiguration besitzen.

Falls im Einzelfall nicht anders angegeben, kann Alkyl geradkettig oder verzweigt sein. Entsprechendes gilt für davon abgeleitete Reste, wie Alkoxy, Alkylthio, Alkylamino, Dialkylamino, Aralkyl oder Alkanoyl.

Unter Cycloalkyl werden auch alkylsubstituierte Reste, wie z.B. 4-Methylcyclohexyl oder 2,3-Dimethylcyclopentyl verstanden.

$(C_6-C_{14})$-Aryl ist beispielsweise Phenyl, Naphthyl, Biphenylyl oder Fluorenyl; bevorzugt ist Phenyl. Entsprechendes gilt für davon abgeleitete Reste, wie z.B. Aryloxy, Aroyl, Aralkyl und Aralkyloxy. Bevorzugte Aralkylreste sind Benzyl und Phenethyl. Halogen bedeutet Fluor, Chlor, Brom oder Jod, vorzugsweise Chlor oder Brom.

Falls nicht anders angegeben, steht die Abkürzung eines Aminosäurerestes ohne einen Stereodeskriptor für den Rest in der L-Form (vgl. Schröder, Lübke, The Peptides, Band I, New York 1965, Seiten xiiixxix).

Als Rest eines heterocyclischen Ringsystems der Formel IV kommen insbesondere Reste von Heterocyclen aus der folgenden Gruppe in Betracht:

Pyrrolidin (A); Piperidin (B); Tetrahydroisochinolin (C); Decahydroisochinolin (D); Octahydroindol (E); Octahydrocyclopenta[b]pyrrol (F); 2-Aza-bicyclo[2.2.2]octan (G); 2-Azabicyclo[2.2.1]heptan (H); 2-Azaspiro-[4.5]decan (I); 2-Azaspiro[4.4]nonan (J); Spiro[(bicyclo[2.2.1]heptan)-2,3-pyrrolidin] (K); Spiro[(bicyclo[2.2.2]-octan)-2,3-pyrrolidin] (L); 2-Azatricyclo[4.3.0.1$^{6,9}$]decan (M); Decahydrocyclohepta[b]pyrrol (N); Octahydroisoindol (O); Octahydrocyclopenta[c]pyrrol (P); 2,3,3a,4,5,7a-Hexahydroindol (Q); Tetrahydrothiazol (R); 2-Azabicyclo[3.1.0]hexan (S); die alle gegebenenfalls substituiert sein können.

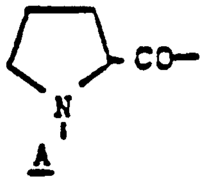 A 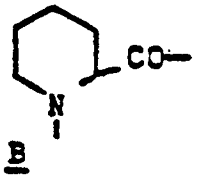 B 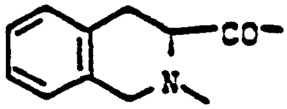 C 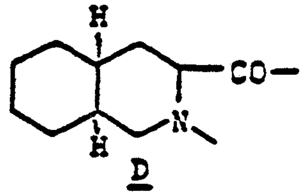 D

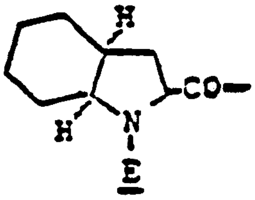 E 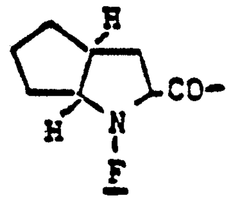 F 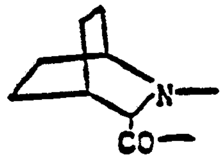 G

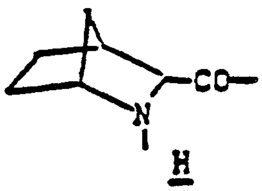 H 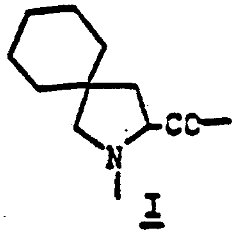 I 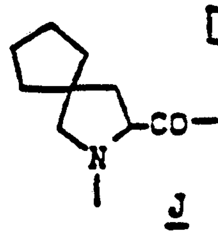 J 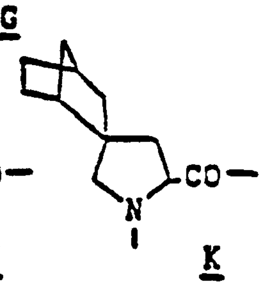 K

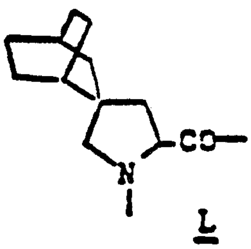 L 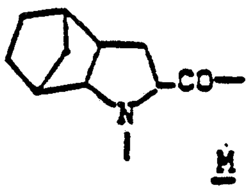 M 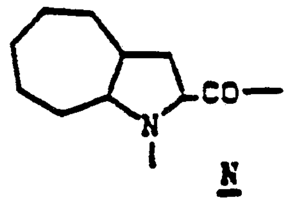 N 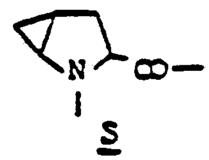 S

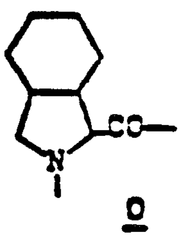 O 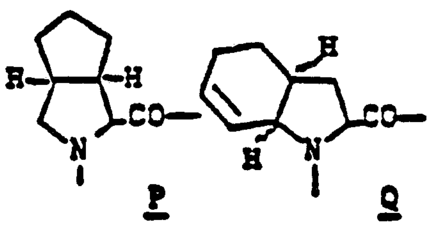 P Q 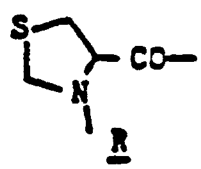 R

Y ist tert.-Butyl oder ein mit in der Kohlenhydratchemie üblichen Schutzgruppen geschützter, teilgeschützter oder ungeschützter Glycosylrest, der sich von einer Glycopyranose, Glycofuranose oder einem Oligosaccharid ableitet.

Bevorzugt sind geschützte Glycosylreste. Die Glycosylreste können sowohl α- als auch β-glycosidisch mit dem Serin-Rest verknüpft sein.

Y kann beispielsweise ein Glycofuranosyl- oder Glycopyranosyl-Rest sein, der sich von natürlich vorkommenden Aldotetrosen, Aldopentosen, Aldohexosen, Ketopentosen, Desoxyaldosen, Aminoaldosen und Oligosacchariden, wie Di- und Trisacchariden, sowie deren Stereoisomeren ableiten.

Die Glycosylreste Y leiten sich insbesondere von natürlichen, im Mikroorganismen, Pflanzen, Tieren oder Menschen vorkommenden D- oder L-Monosacchariden wie Ribose (Rib), Arabinose (Ara), Xylose (Xyl), Lyxose (Lyx), Allose (All), Altrose (Alt), Glucose (Glc), Mannose (Man), Gulose (Gul), Idose (Ido), Galactose (Gal), Talose (Tal), Erythrose (Ery), Threose (Thr), Psicose (Psi), Fructose (Fru), Sorbose (Sor), Tagatose (Tag), Xylulose (Xyu), Fucose (Fuc), Rhamnose (Rha), Olivose (Oli), Oliose (Olo), Mycarose (Myc), Rhodosamin (RN), N-Acetyl-glucosamin (GlcNAc), N-Acetyl-galactosamin (GalNAc), N-Acetyl-mannosamin (ManNAc) oder Disacchariden, wie Maltose (Mal), Lactose (Lac), Cellobiose (Cel), Gentiobiose (Gen), N-Acetyl-lactosamin (LacNAc), Chitobiose (Chit), β-Galactopyranosyl(1→3)-N-acetylgalactosamin und β-Galactopyranosyl-(1→3)- oder -(1→4)-N-acetyl-glucosamin, sowie deren synthetischen Derivaten, wie 2-Desoxy-, 2-Amino-2-Acetamido- oder 2-Halogeno-, bevorzugt Bromo- und Jodo-Zucker ab.

Unter den in der Kohlenhydratchemie üblichen Schutzgruppen versteht man z.B. die ($C_1$-$C_{10}$)-Acylschutzgruppen wie ($C_1$-$C_6$)-Alkanoyl (z.B. Acetyl-, Trichloracetyl-, Trifluoracetyl-), Benzoyl- oder p-Nitrobenzoyl-, sowie gegebenenfalls modifizierte Methyl-, Methyloxymethyl-, Benzyl-, Tetrahydropyranyl-, Benzyliden-, Isopropyliden- oder Trityl-Gruppen, wobei hier die Acylschutzgruppen, insbesondere die Acetyl- (Ac-)Gruppe, bevorzugt sind.

Bevorzugt sind solche Reste A, $A^1$, $A^2$, B, C, D, E, F, G, X, V, Z, $R^N$, $L'$, N, $B^2$ und $R^C$, die sich von natürlich vorkommenden Aminosäuren (s. z. B. Schröder, Lübke, The Peptides, Volume I, New York 1965, Seiten 137-270), deren Antipoden oder deren einfachen Metaboliten ableiten.

Eine bevorzugte Ausführungsform ist dadurch gekennzeichnet, daß Verbindungen der Formel I angewendet werden, in der

a) X Wasserstoff, ($C_1$-$C_5$)Alkoxycarbonyl, ($C_6$-$C_{12}$)-Aryloxycarbonyl, ($C_7$-$C_{13}$)-Aralkyloxycarbonyl, ($C_1$-$C_6$)-Alkanoyl, ($C_7$-$C_{13}$)-Aroyl, Arginyl, Lysyl oder Arginyl-Arginyl bedeutet, X fehlen kann, gegebenenfalls in $\alpha$-Stellung verzweigtes und gegebenenfalls in $\omega$-Stellung durch Amino oder Guanidino monosubstituiertes ($C_1$-$C_{12}$)-Alkylcarbonyl oder ($C_3$-$C_8$)-Cycloalkylcarbonyl bedeuten oder für Ser, Thr oder Q, jeweils in ihrer L- bzw. D-Konfiguration oder Ser-Ser, Thr-Thr, Ser-Thr, Pro-Ser, Pro-Thr, Thr-Pro oder Ser-Pro, vorzugsweise Ser-Ser steht, wobei jede Aminosäure in ihrer L- bzw. D-Konfiguration vorliegen kann und die N-terminale Aminogruppe der Aminosäure oder des Dipeptid-Restes frei vorliegt oder durch ($C_1$-$C_5$)-Alkoxycarbonyl, ($C_6$-$C_{12}$)-Aryloxycarbonyl, ($C_7$-$C_{13}$)-Aralkyloxycarbonyl, ($C_1$-$C_6$)-Alkanoyl, ($C_7$-$C_{13}$)-Aroyl, Arginyl, Lysyl, $\epsilon$-Aminocapropyl oder Arginyl-Arginyl acyliert ist;

V Cys oder, falls X fehlt, Mercaptopropionsäure, Mercaptoessigsäure oder Mercaptobuttersäure bedeutet;

A Phe, Tyr(Me), Tyr($Bu^t$), 4-Halogenphenylalanin oder einen L-2-Thienylalanin-Rest, vorzugsweise Phe bedeutet;

$A^1$ Gly, Ala oder D-Ala bedeutet;

$A^2$ Gly, Ala oder D-Ala bedeutet; oder

$A^1$ und $A^2$ zusammen gegebenenfalls verzweigtes $\omega$-Amino-($C_3$-$C_8$)-alkylcarbonyl bedeutet;

B Arg oder Lys bedeutet;

C Ile, Phe, Leu, Val, Cyclohexylalanin-, tert.-Butylglycin-, Neopentylglycin- oder einen L-2-Thienylalanin-Rest bedeutet;

D Asp, Glu, Gln, Asn, Leu, Ile, Trp, Asp($OBu^t$), Glu($OBu^t$), Glu(OBzl) oder einen 2-Thienylalanin-Rest, vorzugsweise Asp, Glu oder Leu bedeutet, wobei die Aminosäuren jeweils in ihrer L- oder D-Konfiguration vorliegen können;

E Gln, Thr oder Pro bedeutet;

F Ser, Thr oder Ala, vorzugsweise Ser oder Ala, jeweils in ihrer L- oder D-Konfiguration bedeutet;

G Gly, Ala oder D-Ala bedeutet

und

Z für ein Rest der Formel II steht,

worin

H Asn, Ser, Pro oder Thr, vorzugsweise Pro, Thr oder Asn, jeweils in ihrer L- oder D-Form, oder Gly bedeutet;

I Ser, Thr, Ala oder Ser($Bu^t$), vorzugsweise Ser, jeweils in ihrer L- oder D-Form bedeutet;

J Phe, Tyr(Me), Cyclohexylalanyl oder einen L-2-Thienylalanin-Rest, vorzugsweise Phe bedeutet;

K Arg, Lys, Orn oder eine Bindung bedeutet;

L Tyr, Q, Gly, Tyr(Me) oder eine Bindung bedeutet;

M Arg-OH, Arg-$NH_2$, OH, OR, $NH_2$, $NHR'$, Gly-Lys-Arg-OH oder Gly-Lys-Arg-$NH_2$ bedeutet;

R unverzweigtes ($C_1$-$C_6$)-Alkyl bedeutet

und

$R'$ -$[CH_2]_n$-$NH_2$ oder -$[CH_2]_n$-NH-C(NH)$NH_2$, wobei n ganzzahlig ist und für 3-8 steht, bedeutet,

oder

b) X-$\overset{3}{V}$-A-$A^1$-$A^2$- und Gly-Ala-E-F-G-Leu-Gly-$\overset{19}{Cys}$-Z fehlen und der Rest -B-C-D-B-C- für Verbindungen der Formel Ia steht, in welcher

$L'$ für den Rest von Isoleucin, Valin, Threonin, Serin, O-($C_1$-$C_9$)-Alkylthreonin, O-($C_1$-$C_9$)-Alkylserin, Leucin, Prolin oder des $\omega$-($C_1$-$C_6$)-Alkyl- vorzugsweise tert. Butylesters von Glutaminsäure oder Asparaginsäure steht;

N für den Rest von Valin, Isoleucin, Leucin, Phenylalanin, Tryptophan, gegebenenfalls O-($C_1$-$C_6$)-alkyliertem Tyrosin, Glutamin, Asparagin, Glutaminsäure-$\gamma$-($C_1$-$C_6$)-alkylester oder Asparaginsäure-$\beta$-($C_1$-$C_6$)-alkylester oder $\epsilon$-Acyl-lysin steht,

und

$B^2$ Arg, D-Arg, Lys oder D-Lys bedeutet, oder deren physiologisch verträgliche Salze.

Besonders bevorzugt ist eine Ausführungsform, die dadurch gekennzeichnet ist, daß Verbindungen der Formel I angewendet werden, in der

X Wasserstoff, Arginyl oder Arginyl-Arginyl, Ser, Q oder Ser-Ser bedeutet, wobei Ser und Q jeweils in der L- oder D-Konfiguration vorliegen können und die N-terminale Aminogruppe des Aminosäure- oder Dipeptid-Restes frei vorliegt oder durch Arginyl oder Arginyl-Arginyl acyliert ist oder X fehlen kann;

V Cys oder, falls X fehlt, Mercaptopropionsäure bedeutet;

A Phe bedeutet;

B Arg oder Lys bedeutet;

C Ile, Leu, Val oder einen L-2-Thienylalanin-Rest bedeutet;

D Asp, Gln, Leu, Ile, $Asp(OBu^t)$, $Glu(OBu^t)$, $Tyr(Bu^t)$ oder Tyr(Me) bedeutet;

E Gln, Thr oder Pro bedeutet;

F Ser oder Ala jeweils in ihrer L- oder D-Konfiguration bedeutet;

G Gly, Ala, oder D-Ala bedeutet

und

Z für einen Rest der Formel II steht

worin

H Asn, Pro oder Thr, jeweils in ihrer L- oder D-Form, oder Gly bedeutet;

I Ser, Thr oder Ala jeweils in ihrer L- oder D-Form bedeutet;

J Phe, Tyr(Me), Cyclohexylalanyl oder einen 2-Thienylalanin-Rest bedeutet;

K Arg, Lys oder eine Bindung bedeutet;

L Tyr, $Tyr(Bu^t)$ oder eine Bindung bedeutet;

M OH, $NH_2$, $NHR'$, Gly-Lys-Arg-OH oder Gly-Lys-Arg-$NH_2$ bedeutet

und

$R'$ $-[CH_2]_2-NH_2$ oder $-[CH_2]_n-NH-C(NH)NH_2$ bedeutet, wobei n ganzzahlig ist und für 3-8 steht.

Besonders vorteilhaft können die folgenden Verbindungen erfindungsgemäß verwendet werden:

H-Ser-Ser-Cys-Phe-Gly-Gly-Arg-Ile-Glu-Arg-Ile-Gly-Ala-

-Gln-Ser-Gly-Leu-Gly-Cys-Asn-Ser-Phe-Arg-Tyr-OH

H-Ser-Ser-Cys-Phe-Gly-Gly-Arg-Ile-Asp-Arg-Ile-Gly-Ala-Gln-

-Ser-Gly-Leu-Gly-Cys-Asn-D-Ser-Phe-Arg-Tyr-OH

H-Ser-Ser-Cys-Phe-Gly-Gly-Arg-Ile-Asp-Arg-Ile-Gly-Ala-Gln-

-D-Ala-Gly-Leu-Gly-Cys-Asn-Ser-Phe-Arg-Tyr-OH

H-Ser-Ser-Cys-Phe-Gly-Gly-Arg-Ile-Asp-Arg-Ile-Gly-Ala-Gln-

-Ser-D-Ala-Leu-Gly-Cys-Asn-Ser-Phe-Arg-Tyr-OH

H-Ser-Ser-Cys-Phe-Gly-Gly-Arg-Ile-Asp-Arg-Ile-Gly-Ala-Gln-

-Ser-Gly-Leu-Gly-Cys-Asn-Ser-Phe-Lys-$NH_2$

H-Arg-Arg-Ser-Ser-Cys-Phe-Gly-Gly-Arg-Ile-Glu-Arg-Ile-

-Gly-Ala-Gln-Ser-D-Ala-Leu-Gly-Cys-Asn-Ser-Phe-Arg-

-Tyr-OH

H-Ser-Ser-Cys-Phe-Gly-Gly-Arg-Ile-Glu-Arg-Ile-Gly-Ala-

-Gln-Ser-Gly-Leu-Gly-Cys-Asn-Ser-Phe-Arg-$NH_2$

H-Arg-Arg-Ser-Ser-Cys-Phe-Gly-Gly-Arg-Ile-Glu-Arg-Ile-

-Gly-Ala-Gln-Ser-Gly-Leu-Gly-Cys-Asn-Ser-Phe-Arg-$NH_2$

H-Ser-Ser-Cys-Phe-Gly-Gly-Arg-Ile-Glu-Arg-Ile-Gly-Ala-

-Gln-Ser-Gly-Leu-Gly-Cys-Thr-Ser-Phe-Arg-$NH_2$

H-Ser-Ser-Cys-Phe-Gly-Gly-Arg-Ile-Glu-Arg-Ile-Gly-Ala-

-Gln-Ser-Gly-Leu-Gly-Cys-Asn-Ser-Phe-Arg-Tyr-Gly-Lys-

-Arg-OH

H-Ser-Ser-Cys-Phe-Gly-Gly-Arg-Ile-Asp-Arg-Ile-Gly-Ala-

-Gln-Ser-Gly-Leu-Gly-Cys-Asn-Ser-Phe-Arg-Tyr-Gly-Lys-

-Arg-OH

H-Ser-Ser-Cys-Phe-Gly-Gly-Arg-Ile-Glu-Arg-Ile-Gly-Ala-

-Thr-Ser-Gly-Leu-Gly-Cys-Pro-Ser-Phe-Arg-Tyr-OH

H-Ser-Ser-Cys-Phe-Gly-Gly-Arg-Ile-Glu-Arg-Ile-Gly-Ala-

-Thr-Ser-Gly-Leu-Gly-Cys-Pro-Ser-Phe-Arg-Tyr-Gly-Lys-Arg-OH

H-Ser-Ser-Cys-Phe-Gly-Gly-Arg-Ile-Asp-Arg-Ile-Gly-Ala-

-Gln-Ser-Gly-Leu-Gly-Cys-Asn-Ser-Phe-NH-$(CH_2)_4$-$NH_4$

H-Ser-Ser-Cys-Phe-Gly-Gly-Arg-Ile-Asp-Arg-Ile-Gly-Ala-

-Gln-Ser-Gly-Leu-Gly-Cys-Asn-Ser-Phe-NH-$(CH_2)_6$-$NH_2$

H-Ser-Ser-Cys-Phe-Gly-Gly-Arg-Ile-Asp-Arg-Ile-Gly-Ala-

-Gln-Ser-Gly-Leu-Gly-Cys-Asn-Ser-Phe-NH-$(CH_2)_8$-$NH_2$

H-Aoc-Ser-Cys-Phe-Gly-Gly-Arg-Ile-Asp-Arg-Ile-Gly-Ala-

-Gln-Ser-Gly-Leu-Gly-Cys-Asn-Ser-Phe-Arg-Tyr-OH

H-Iva-Cys-Phe-Gly-Gly-Arg-Ile-Asp-Arg-Ile-Gly-Ala-Gln-Ser-

-Gly-Leu-Gly-Cys-Asn-Ser-Phe-Arg-Tyr-OH

H-Aoc-Cys-Phe-Gly-Gly-Arg-Ile-Asp-Arg-Ile-Gly-Ala-Gln-Ser-

-Gly-Leu-Gly-Cys-Asn-Ser-Phe-Arg-Tyr-OH

H-Arg-Arg-Ser-Ser-Cys-Phe-Gly-Gly-Arg-Ile-Asp-Arg-Ile-

-Gly-Ala-Gln-Ser-Gly-Leu-Gly-Cys-Asn-Ser-Phe-Arg-Gly-OH

H-Cys-Phe-D-Ala-Gly-Arg-Ile-Asp-Arg-Ile-Gly-Ala-Gln-Ser-

-Gly-Leu-Gly-Cys-Asn-Ser-Phe-NH-$(CH_2)_4$-$NH_2$

H-Ser-Ser-Cys-Tyr(Me)-Gly-Gly-Arg-Ile-Asp-Arg-Ile-

-Gly-Ala-Gln-Ser-Gly-Leu-Gly-Cys-Asn-Ser-Phe-Arg-Tyr-OH

H-Ser-Ser-Cys-Phe-Gly-Gly-Arg-Ile-Asp-Arg-Ile-Gly-Ala-

-Gln-Ser-Gly-Leu-Gly-Cys-Asn-Ser-Phe-Arg-Tyr-OH

H-Ser-Ser-Cys-Phe-Gly-Gly-Arg-Met-Asp-Arg-Ile-Gly-Ala-

-Gln-Ser-Gly-Leu-Gly-Cys-Asn-Ser-Phe-Arg-Tyr-OH

H-Ser-Ser-Cys-Phe-NH-$(CH_2)_5$-CO-Arg-Ile-Asp-Arg-Ile-Gly-

-Ala-Gln-Ser-Gly-Leu-Gly-Cys-Asn-Ser-Phe-Arg-Tyr-OH

H-Aoc-Cys-Phe-D-Ala-Gly-Arg-Ile-Asp-Arg-Ile-Gly-Ala-

-Gln-Ser-Gly-Leu-Gly-Cys-Asn-Ser-Phe-Lys-$NH_2$

H-D-Aoc-Cys-Phe-D-Ala-Gly-Arg-Ile-Asp-Arg-Ile-Gly-Ala-

-Gln-Ser-Gly-Leu-Gly-Cys-Asn-Ser-Phe-Lys-$NH_2$

H-Aoc-Cys-Phe-D-Ala-Gly-Arg-Ile-Asp-Arg-Ile-Gly-Ala-

Gln-Ser-Gly-Leu-Gly-Cys-Asn-Ser-Phe-Arg-Aoc-$NH_2$

H-Aoc-Cys-Phe-D-Ala-Gly-Arg-Ile-Asp-Arg-tert.Butylglycyl-
Gly-Ala-Gln-Ser-Gly-Leu-Gly-Cys-Asn-Ser-Phe-Arg-$NH_2$

H-Aoc-Cys-Phe-D-Ala-Gly-Arg-Ile-Asp-Arg-Neopentylglycyl-
Gly-Ala-Gln-Ser-Gly-Leu-Gly-Cys-Asn-Ser-Phe-Arg-$NH_2$

H-Cys-Phe-D-Ala-Gly-Arg-Ile-Glu-Arg-Ile-Gly-Ala-Thr-
Ser-Gly-Leu-Gly-Cys-Pro-Ser-Phe-NH-$(CH_2)_4$-$NH_2$

H-Aoc-Ser-Cys-Phe-Gly-Gly-Arg-Ile-Asp-Arg-Ile-Gly-Ala-
Gln-Ser-Gly-Leu-Gly-Cys-Asn-Ser-Phe-NH-$(CH_2)_8$-$NH_2$

H-Cys-Phe-Gly-Gly-Arg-Ile-Asp-Arg-Ile-Gly-Ala-Gln-Ser-
Gly-Leu-Gly-Cys-Asn-Ser-Phe-NH-$(CH_2)_8$-$NH_2$

H-Cys-Phe-D-Ala-Gly-Arg-Ile-Asp-Arg-Ile-Gly-Ala-Gln-
Ser-Gly-Leu-Gly-Cys-Asn-Ser-Phe-NH-$(CH_2)_6$-$NH_2$

H-Aoc-Cys-Phe-D-Ala-Gly-Arg-Ile-Glu-Arg-Ile-Gly-Ala-
Thr-Ser-Gly-Leu-Gly-Cys-Gly-Ser-Phe-NH-$(CH_2)_6$-$NH_2$

H-Arg-Ile-Asp(OtBu)-Arg-Ile-OH

H-D-Arg-Ile-D-Leu-Arg-Ile-OH
H-Lys-Ile-Asp(OtBu)-Arg-Ile-$NH_2$
H-Arg-Trp-Asp(OtBu)-Arg-Phe-$NH_2$
cyclo-(D-Arg-Ile-D-Leu-Arg-Ile)

Diese Verbindungen lassen sich z. B. nach den in den deutschen Patentanmeldungen P 36 14 833.4 (EP-A 231 752), P 37 23 551.6 oder P 36 19 633.9 (EP-A 249 169) beschriebenen Verfahren durch stufenweisen Aufbau einzelner Aminosäuren oder Fragmente, wobei anschließend gegebenenfalls eine oder mehrere zum Schutz anderer funktioneller Gruppen temporär eingeführte Schutzgruppen abgespalten, eine Disulfidbrücke zwischen den beiden Cys-Resten (falls vorhanden) geknüpft und die so erhaltenen Verbindungen der Formel I gegebenenfalls in ihre physiologisch verträglichen Salze überführt werden, herstellen.

Die Verbindungen der Formel I (ANF, dessen Teilsequenzen und Analoga) haben natriuretische, diuretische und vasoaktive Wirkung (Currie et al., Science 223, 67, 1984; Kangawa, Matsuo, Biochem. Biophys. Res. Commun. 118, 131, 1984; DE 36 14 833.4; DE 37 23 551.6; DE 36 19 633.9).

Die Verbindungen der Formel I sind z. B. bekannt aus EP 189 084, EP 152 333, US-A 4,609,725, EP 166 585, EP 164 273, EP 155 786, EP 153 865, EP 147 193, J 61 286 400-A, J 61 236 698-A2, EP 306 085, EP 173 384, EP 116 784, EP 142 487, EP 162 032, US 4,496,544, US 4,557,864, EP 140 731, EP 156 786, WO 85/04872, WO 85/04870, EP 180 615, EP 223 143, WO 85/02850, J 61 243 100-A, EP 206 769, EP 207 044, J 61 197 597-A, EP 87 100 109.5, EP 87 108 184.0 und DE 37 23 551.6.

Vorteilhaft sind auch folgende ANF-Analoge (Nomenklatur siehe The New England Journal of Medicine, Vol. 316, No. 20 (1987) 1278 - 1279):
L 346 670 (MSD), L 364 343 (MSD), H-ANP (Cal. Biotech.), alpha-ANP (Suntory), ANP III (Monsanto), PL 058 (Peninsula) sowie rat [$Mac^{105}$] ANF (101-126), rat [$Mpr^{105}$] ANF (101-126), rat [$Mbu^{105}$] ANF (101-126) (P.W. Schiller et al., Biochem. Biophys. Res. Comm. 143 (1987) 499).

Die erfindungsgemäßen Verbindungen verbessern arrhythmische Zustände, die Hämodynamik und den Stoffwechsel des Herzens. Ihre Wirkung wurde an Ratten im Test nach Langendorff geprüft (O. Langendorff "Untersuchungen am überlebenden Säugetierherzen", Pflügers Archiv 1985, Bd. 61, 291 - 332; H. Bardenheuer, J. Schrader "Relationships between myocardial oxygen consumption, coronary flow and adenosin release in an improved isolated working heart preparation of guinea pigs", Circulation Research, 1983, Bd. 51, 263 - 271).

**METHODE**

Die Koronargefäße der isolierten arbeitenden Rattenherzen wurden retrograd über die Aorta mit Krebs-Henseleit-Lösung perfundiert. In den linken Ventrikel wurde ein Ballon verbracht, der mit einem künstlichen "systemischen" Kreislauf verbunden war. Die Druck-Volumenarbeit wurde erreicht, indem aus dem Ballon in den systemischen Kreislauf Kochsalzlösung gepumpt wurde. Die Herzen wurden mit einem Druck von 65 mmHg perfundiert, die Nachlast betrug ebenfalls 65 mmHg und die Vorlast 7,4 mmHg. In einer Kontrollperfusionsperiode wurden die Herzen über 20 Minuten perfundiert. Danach wurde die linke Koronararterie in der Nähe ihres Austritts aus der Aorta mit einem Clip verschlossen. Nach 15 Minuten (ischämische Periode) wurde der Verschluß geöffnet und es schloß sich eine 30 minütige Reperfusionsphase an. Während dieses experimentellen Zeitverlaufs untersuchten wir die Effekte des ANF auf kardiodynamische Parameter, wie linker Ventrikeldruck (LVD), dp/dt max, Herzfrequenz (HF), Koronarfluß (KorF), myokardialer Sauerstoffverbrauch (MSV), auf Enzyme im koronarvenösen Effluat wie Laktat dehydrogenase (LDH) und Kreatinkinase (KK) sowie Laktat, auf metabolische Parameter im Myokard wie Laktat, Glykogen, Adenosintriphosphat (ATP) und Kreatinphosphat (KP) und auf Reperfusionsarrhythmien mittels EKG.

Eine Substanz wird dann als wirksam bezeichnet, wenn nach dem Abklemmen der linken Herzkoronararterie und anschließender Reperfusion keine Arrhythmien - im Vergleich zur unbehandelten Kontrolle - entstehen, wobei das Kammerflimmern (KF) einen meßbaren Parameter darstellt.

Die erfindungsgemäßen Verbindungen wurden in den entsprechenden Konzentrationen vor und nach Okklusion der linken Koronararterie perfundiert (n:10); (n:4). Kontrollherzen wurden mit Krebs-Henseleit-Lösung perfundiert (n:10).
Die Ergebnisse sind in den nachfolgenden Tabellen 1 bis 3 dargestellt.

Tabelle 1: human ANF-(103-126)/Konzentration: [$10^{-7}$ mol/l]

| | Kontrollperfusionsperiode | | Ischämische Periode | | Reperfusionsperiode | |
|---|---|---|---|---|---|---|
| | Kontrolle (n : 10) | h-ANF( 103-126) (n : 10) | Kontrolle (n : 10) | h-ANF(103-126) (n : 10) | Kontrolle (n : 10) | h-ANF(103-126) (n : 10) |
| LVD [mmHg] | 66 ± 2 | * 55 ± 2 | 22 ± 2 | * 45 ± 3 | 52 ± 4 | * 37 ± 2 |
| LVdp/$dt_{max}$ [mmHg • $sec^{-1}$] | 2256 ± 98 | * 2125 ± 91 | 638 ± 91 | * 1663 ± 117 | 1821 ± 131 | * 1385 ± 91 |
| HF [Schläge/Min] | 302 ± 5 | 298 ± 4 | 298 ± 10 | 301 ± 5 | 275 ± 8 | 280 ± 4 |
| KorF [ml/Min/g] | 11,6 ± 0,7 | 11,1 ± 0,2 | 3,9 ± 0,5 | * 9,0 ± 0,2 | 9,9 ± 0,7 | * 11,7 ± 0,3 |
| MSV [ml/min/100 g] | 17,7 ± 1,0 | * 14,5 ± 0,3 | 6,4 ± 0,9 | * 11,5 ± 0,3 | 14,4 ± 0,9 | 14,7 ± 0,4 |
| Laktat [µmol/min/g] | 0,23 ± 0,03 | * 0,13 ± 0,01 | 0,53 ± 0,05 | * 0,18 ± 0,01 | 0,31 ± 0,05 | * 0,12 ± 0,04 |
| KK [mU/min/g] | 37 ± 3 | 28 ± 3 | 70 ± 5 | * 30 ± 2 | 43 ± 8 | * 18 ± 2 |
| LDH [mU/min/g] | 32 ± 3 | 21 ± 2 | 56 ± 3 | * 20 ± 2 | 28 ± 5 | * 12 ± 1 |
| KF [Min] | | | | | 15,0 ± 2,5 | * 2,7 ± 0,9 |

* $p < 0,05$

Tabelle 2: cyclo-(D-Arg-Ile-D-Leu-Arg-Ile); Konzentration: [$10^{-7}$ mol/l]

| | Kontrollperfusionsperiode | | Ischämische Periode | | Reperfusionsperiode | |
|---|---|---|---|---|---|---|
| | Kontrolle (n : 10) | cyclo-(D-Arg-Ile-D-Leu-Arg-Ile) (n : 4) | Kontrolle (n : 10) | cyclo-(D-Arg-Ile-D-Leu-Arg-Ile) (n : 4) | Kontrolle (n : 10) | cyclo-(D-Arg-Ile-D-Leu-Arg-Ile) (n : 4) |
| LVD [mmHg] | 66 ± 2 | 68 ± 3 | 22 ± 2 | * 49 ± 4 | 52 ± 4 | 48 ± 6 |
| $LVdp/dt_{max}$ [mmHg · $sec^{-1}$] | 2256 ± 98 | 2589 ± 101 | 638 ± 91 | * 1784 ± 109 | 1821 ± 131 | 1724 ± 111 |
| HF [Schläge/Min] | 302 ± 5 | 306 ± 7 | 298 ± 10 | 299 ± 7 | 275 ± 8 | 284 ± 10 |
| KorF [ml/Min/g] | 11,6 ± 0,7 | 12,3 ± 0,8 | 3,9 ± 0,5 | * 8,7 ± 0,4 | 9,9 ± 0,7 | * 12,9 ± 0,9 |
| Laktat [µmol/min/g] | 0,23 ± 0,02 | * 0,18 ± 0,04 | 0,53 ± 0,05 | * 0,24 ± 0,03 | 0,31 ± 0,05 | * 0,16 ± 0,05 |
| KK [mU/min/g] | 37 ± 3 | 32 ± 4 | 70 ± 5 | * 32 ± 5 | 43 ± 8 | * 20 ± 4 |
| LDH [mU/min/g] | 32 ± 3 | 29 ± 5 | 56 ± 3 | * 22 ± 4 | 28 ± 5 | * 14 ± 3 |

* $p < 0,05$

Tabelle 3: Myokardiale Gewebsspiegel [µmol/g Trockengewicht] von Laktat, Glykogen, ATP und KP in mit h-ANF (103-126) oder cyclo-(D-Arg-Ile-D-Leu-Arg-Ile) [$10^{-7}$ mol/l] behandelten ischämischen, isolierten arbeitenden Rattenherzen perfundiert für 65 Minuten.

| | Kontrolle (n : 10) | | h-ANF (103-126) (n : 10) | | cyclo-(D-Arg-Ile-D-Leu-Arg-Ile) (n : 4) |
|---|---|---|---|---|---|
| Laktat | 17,74 ± 1,96 | * | 4,03 ± 0,62 | * | 3,87 ± 0,46 |
| Glykogen | 88,36 ± 3,44 | * | 113,15 ± 3,74 | * | 121,09 ± 2,97 |
| ATP | 5,31 ± 0,48 | * | 11,01 ± 0,59 | * | 13,25 ± 0,41 |
| KP | 6,02 ± 0,36 | * | 13,89 ± 0,99 | * | 15,22 ± 0,83 |

* $p < 0,01$

Die erfindungsgemäßen Verbindungen sind aufgrund ihrer pharmakologischen Eigenschaften neben der Anwendung als Diuretikum, Salidiuretikum, vasorelaxierendes oder immunmodulierendes Mittel auch für die Prophylaxe bei ischämischen Herzzuständen und für die Behandlung von Herzerkrankungen, wie beispielsweise akuter Herzinsuffizienz, ischämischer Zustände des Herzens bis hin zum Herzinfarkt oder zur Prävention gegen Reperfusionsarrhythmien geeignet.

Die Erfindung betrifft daher weiter die Anwendung der erfindungsgemäßen Verbindungen bei der Prophylaxe bei ischämischen Herzzuständen und bei der Behandlung von Herzerkrankungen, insbesondere der akuten Herzinsuffizienz, ischämischer Zustände des Herzens und der Prävention gegen Reperfusionsarrhythmien.

Die Erfindung umfaßt weiterhin die genannten Verbindungen enthaltenden Arzneimittel, Verfahren zu deren Herstellung sowie die Verwendung der erfindungsgemäßen Verbindungen bei der Herstellung von Arzneimitteln, die zur Prophylaxe und Behandlung der vorstehend genannten Krankheiten eingesetzt werden.

In Ausübung der erfindungsgemäßen Methode können die oben beschriebenen ANF, dessen Teilsequenzen und Analoga an Säugern wie Hunden, Katzen, Ratten, Meerschweinchen, Menschen usw. angewendet werden.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfsstoffen, gegebenenfalls als Retardzubereitung in Form von Tabletten, Kapseln, Pastillen, Implantaten, Rods, Mikrokapseln, Transdermalen Therapeutischen Systemen (TTS), Emulsionen, Suspensionen, Lösungen oder Sprays eingesetzt, wobei der Wirkstoffgehalt $10^{-4}$ bis $10^{-12}$ mol/l, bevorzugt $10^{-7}$ bis $10^{-8}$ mol/l beträgt. Die Dosierung des zu verabreichenden Wirkstoffs der Formel I und die Häufigkeit der Verabreichung hängen

von der Wirkstärke und Wirkdauer der verwendeten Verbindung ab; außerdem auch von Art und Stärke der zu behandelnden Krankheit sowie von Geschlecht, Alter, Gewicht und individueller Ansprechbarkeit des zu behandelnden Säugers.

Welche Hilfsstoffe für die gewünschte Arzneimittelformulierung geeignet sind, ist dem Fachmann aufgrund seines Fachwissens geläufig. Neben Lösemitteln, Gelbildnern, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können beispielsweise auf die Schleimhäute (intranasal, buccal) oder parenteral (intravenös, intraarteriell, intracardial, subcutan, percutan) appliziert werden, wobei die parenterale Applikation bevorzugt ist.

Für eine subcutane, intraarterielle, intravenöse oder intracardiale Anwendungsform werden die aktiven Verbindungen oder deren physiologisch verträgliche Salze, gewünschtenfalls mit den dafür üblichen Substanzen wie Lösungsvermittler, Emulgatoren oder weiteren Hilfsstoffen in Lösung, Suspension oder Emulsion gebracht, gegebenenfalls anschließend lyophilisiert und vor Applikation das Lyophilisat in Aqua pro injectione, das einen physiologisch verträglichen Puffer und gegebenenfalls ein übliches Isotoniemittel enthält, gelöst. Als Lösungsmittel kommen z. B. in Frage Wasser, physiologische Kochsalzlösung oder Alkohole, z. B. Ethanol, Propanol, Glycerin, daneben auch Zuckerlösungen wie Glucose- oder Mannitlösungen oder auch eine Mischung aus den verschiedenen genannten Lösungsmitteln.

Zur Applikation auf die Schleimhäute werden die aktiven Verbindungen mit den dafür geeigneten Zusatzstoffen wie Trägerstoffen, Stabilisatoren oder inerten Verdünnungsmitteln vermischt und durch die üblichen Methoden in geeignete Darreichungsformen gebracht wie Tabletten, Pastillen, wäßrige alkoholische oder ölige Suspensionen oder wäßrige, alkoholische oder ölige Lösungen. Als inerte Träger können z. B. Gummi arabicum, Magnesia, Magnesiumcarbonat, Milchzucker, Glucose oder Stärke, insbesondere Maisstärke verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht wie Sonnenblumenöl oder Lebertran.

Als pharmazeutische Formulierungen für die Verabreichung in Form von Sprays sind geeignet z. B. Lösungen, Suspensionen oder Emulsionen des erfindungsgemäßen Wirkstoffs der allgemeinen Formel I in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten.

Verwendet werden können für intranasale Applikation:

1. Wäßrige bzw. wäßrig-alkoholische Lösungen zur Applikation mit einer Tropfpipette bzw. einer Kunststoffquetschflasche oder zur Vernebelung mit einer Dosierzerstäuberpumpe

Die Zubereitung kann neben dem Wirkstoff einen isotonisierenden Zusatz, z. B. Natriumchlorid oder Kaliumnitrat, Glucose, Mannitol oder Dextrane, Puffersubstanzen, z. B. Natrium- oder Kaliumphosphate, Citronensäure und ihre Salze sowie Mischungen von Phosphaten und Citraten im pH-Bereich 3 - 8, ein Konservierungsmittel, z. B. Benzalkoniumchlorid, Benzylalkohol, Quecksilberphenylborat, einen Komplexbildner, z. B. Natriumedetat und als Lösungsmittel Wasser oder Mischungen von Wasser mit ($C_2$-$C_3$)-Alkanolen enthalten. Die Lösung wird mit einem geeigneten Gerät appliziert bzw. in die Nase gesprüht.

2. Wäßrige bzw. wäßrig-alkoholische Gele zum Einbringen in die Nase

Zusätzlich zu 1. enthält ein Gel einen die Viskosität erhöhenden Zusatz, z. B. ein Polyacrylatpolymer oder Celluloseether wie Hydroxypropylmethylcellulose (HPMC), Hydroxyethylcellulose (HEC), Methylhydroxyethylcellulose (MHEC), Carboxymethylcellulose (CMC).

3. Suspensionen in Treibgasen

Die Zubereitung kann neben dem mikronisierten Wirkstoff einen Fluorkohlenwasserstoff. z. B. ⁽ᴿ⁾Frigen F 113 als Suspensionsflüssigkeit und ein Suspendierhilfsmittel, z. B. Sorbitantrioleat oder Sojalecithin enthalten. Als Treibgase eignen sich Fluorkohlenwasserstoffe, z. B. ⁽ᴿ⁾Frigen F 12 und ⁽ᴿ⁾Frigen F 114 bzw. ihre Gemische mit ⁽ᴿ⁾Frigen F 11. Die Abfüllung erfolgt in an sich bekannter Weise nach dem Kälteabfüllverfahren oder aber durch Druckfüllung.

4. Verreibung mit Trägerstoffen, abgefüllt in Hartgelatinekapseln zur intranasalen Anwendung

Der mikronisierte Wirkstoff wird gegebenenfalls nach Zugabe eines Mittels zur Fließverbesserung, wie z. B. Lactose, in Hartgelatinekapseln abgefüllt. Der Inhalt einer Kapsel wird mit einer Inhalationshilfe, die es erlaubt, das Pulver in einen inhalier- oder insufflierbaren Rauch zu überführen, intranasal appliziert.

Die nachfolgenden Beispiele sollen die Erfindung erläutern, jedoch ohne darauf einzuschränken.

**Beispiel 1: Injektionslösungen**

a) human ANF-(103-126) 0,1 g
Polyhydroxybuttersäureester 2,0 g
Natriumdihydrogenphosphat 2,5 g
Natriumchlorid 4,5 g
Natronlauge q.s. pH 6 Wasser für Injektion ad 1 l
b) human ANF-(103-126) 0,1 g
Natriumchlorid 9,0 g
Essigsäure q.s. pH 4 Wasser für Injektion ad 1 l

**Beispiel 2: Lyophilisate**

a) human ANF-(103-126) 0,1 g
Mannitol 50,0 g
Lactose 100,0 g
Wasser ad 1 l
b) human ANF-(103-126) 0,1 g
Glycin 20,0 g
1 N Salzsäure q.s. pH 4 Wasser ad 1 l

**Beispiel 3: Intranasale Zubereitungen**

a) human ANF-(103-126) 1,00 g
Natriumchlorid 0,80 g
Citronensäure 0,11 g
Natriumcitrat 0,15 g
Benzalkoniumchlorid 0,01 g
Dinatriumedetat 0,01 g
Gereinigtes Wasser ad 100,00 g
b) human ANF-(103-126) mikronisiert 1,00 g
Lactose ad 100,00 g

Analog der in den Beispielen beschriebenen Vorschriften wurden Injektionslösungen, Lyophilisate und intranasale Zubereitungen hergestellt, die folgende Wirkstoffe enthalten:
[$Aoc^{104}$] rat ANF-(104-126) rat ANF-(103-126)

[$Aoc^{104}$, D-$Ala^{107}$, $Lys^{125}$] rat ANF-(104-125)amid
[D-$Aoc^{104}$, D-$Ala^{107}$, $Lys^{125}$] rat ANF-(104-125)amid
[$Aoc^{104}$, D-$Ala^{107}$, $Aoc^{126}$] rat ANF-(104-126)amid
cyclo-(D-Arg-Ile-D-Leu-Arg-Ile)-diacetat

## Ansprüche

1. Verwendung von atrionatriuretischem Faktor (ANF), dessen Teilsequenzen oder Analoga zur Herstellung einer pharmazeutischen Zubereitung für die Prophylaxe bei ischämischen Herzzuständen und zur Behandlung von Herzerkrankungen, gekennzeichnet durch einen pharmakologisch wirksamen Gehalt an einer solchen Verbindung in einem pharmazeutisch unbedenklichen Träger.

2. Verwendung gemäß Anspruch 1 zur Herstellung einer Zubereitung gemäß Anspruch 1, dadurch gekennzeichnet, daß eine Verbindung der Formel I

$$\text{X-}\overset{3}{\text{V}}\text{-A-A}^1\text{-A}^2\text{-B-C-D-B-C-Gly-Ala-E-F-G-Leu-Gly-}\overset{19}{\text{Cys}}\text{-Z} \qquad (I)$$

in welcher

a) X Wasserstoff, ($C_1$-$C_5$)-Alkoxycarbonyl, ($C_6$-$C_{12}$)-Aryloxycarbonyl, ($C_7$-$C_{13}$)-Aralkyloxycarbonyl, ($C_1$-$C_6$)-Alkanoyl, ($C_7$-$C_{13}$)-Aroyl, Arginyl, Lysyl, $\epsilon$-Aminocaproyl, Arginyl-Arginyl, Arginyl-Lysyl, Lysyl-Arginyl oder Lysyl-Lysyl bedeutet, X fehlen kann, gegebenenfalls in $\alpha$-Stellung verzweigtes und gegebenenfalls in $\omega$-Stellung durch Amino oder Guanidino monosubstituiertes ($C_1$-$C_{12}$)-Alkylcarbonyl oder ($C_3$-$C_8$)-Cycloalkylcarbonyl bedeutet oder für Ser, Thr, Ser(Y), Thr(Y), Q oder Leu, jeweils in ihrer L-bzw. D-Konfiguration oder Ser-Ser, Thr-Thr, Ser-Thr, Q-Ser, Q-Thr, Thr-Q, Ser-Q, Ser(Y)-Ser oder Ser-Ser(Y) steht, wobei jede Aminosäure in ihrer L-bzw. D-Konfiguration vorliegen kann und die N-terminale Aminogruppe der Aminosäure oder des Dipeptid-Restes frei vorliegt (N-terminaler Rest = H) oder durch ($C_1$-$C_5$)-Alkoxycarbonyl, ($C_6$-$C_{12}$)- Aryloxycarbonyl, ($C_7$-$C_{13}$)-Aralkyloxycarbonyl, ($C_1$-$C_6$)-Alkanoyl, ($C_7$-$C_{13}$)-Aroyl, Arginyl, Lysyl, $\epsilon$-Aminocaproyl, Arginyl-Arginyl, Arginyl-Lysyl, Lysyl-Arginyl oder Lysyl-Lysyl acyliert ist;

V Cys oder, falls X fehlt, $\omega$-Thio($C_2$-$C_8$)alkylcarbonyl, wie beispielsweise Mercaptopropionsäure, Mercaptoessigsäure, Mercaptobuttersäure, bedeutet;

A Phe, Tyr(Me), Tyr($Bu^t$), 4-Halogenphenylalanin, Trp oder einen L-2-Thienylalanin-Rest bedeutet;

$A^1$ Gly,. Ala oder D-Ala bedeutet;

$A^2$ Gly, Ala oder D-Ala bedeutet; oder

$A^1$ und $A^2$ zusammen gegebenenfalls verzweigtes $\omega$-Amino-($C_3$-$C_8$)-alkylcarbonyl bedeutet;

B Arg, Lys, Orn oder einen L-Homoarginin-Rest bedeutet;

C Ile, Met, Phe, Trp, Leu, Ser, Thr, Val, His, Pro, Asn, Ser($Bu^t$), Cyclohexylalanin-, tert. Butylglycin-, Neopentylglycin- oder einen L-2-Thienylalanin-Rest bedeutet;

D Asp, Glu, Gln, Asn, Phe, Leu, Ile, Trp, Pro, Tyr, Ala, Asp($OBu^t$), Asp(OBzl), Glu($OBu^t$), Glu(OBzl), einen 2-Thienylalanin-Rest, Aad, Tyr($Bu^t$) oder Tyr(Me) bedeutet, wobei die Aminosäuren jeweils in ihrer L- oder D-Konfiguration vorliegen können;

E Gln, Thr oder Pro bedeutet;

F Ser, Thr, Pro, Ala, Ser($Bu^t$) oder Thr($Bu^t$), jeweils in ihrer L- oder D-Konfiguration bedeutet;

G Gly, Ala oder D-Ala bedeutet;

Q einen Rest der Formel IV,

$$-\overset{R^1}{\underset{}{N}}-\overset{R^2}{CH}-\overset{O}{\overset{\|}{C}}- \qquad (IV)$$

worin

$R^1$ und $R^2$ zusammen mit den diese Reste tragenden Atomen ein heterocyclisches mono-, bi- oder tricyclisches Ringsystem mit 3 bis 15 C-Atomen bilden,
bedeutet;

Y tert.-Butyl oder einen gegebenenfalls geschützten oder teilgeschützten Glycosylrest bedeutet;

und

Z für einen Rest der Formel II steht,

-H-I-J-K-L-M (II)

worin

H Asn, Ser, Q oder Thr, jeweils in ihrer L- oder D-Form, oder Gly bedeutet;

I Ser, Thr, Ala, Ser($Bu^t$), Thr($Bu^t$) oder Pro, jeweils in ihrer L- oder D-Form bedeuten;

J Phe, Trp, D-Phe, D-Trp, Tyr(Me), Cyclohexylalanyl oder einen 2-Thienylalanin-Rest bedeutet;

K Arg, Lys, Orn oder eine Bindung bedeutet;

L Q, Gly, Tyr, Tyr($Bu^t$), Tyr(Me) oder eine Bindung bedeutet;

M Arg-OH, Arg-$NH_2$, OH, OR, $NH_2$, NHR′, Gly-Lys-Arg-OH, Gly-Lys-Arg-$NH_2$ oder L-Argininol bedeutet;

Q wie oben definiert ist;

R unverzweigtes ($C_1$-$C_6$)-Alkyl bedeutet und

R′ -$[CH_2]_n$-$NH_2$ oder -$[CH_2]_n$-NH-C(NH)$NH_2$, wobei n ganzzahlig ist und für 3-8 steht, bedeutet;

sowie deren physiologisch verträgliche Salze, oder

b) X- $\overset{3}{V}$ -A-A′-$A^2$- und Gly-Ala-E-F-G-Leu-Gly- $\overset{19}{Cys}$ -Z fehlen und der Rest -B-C-D-B-C- für Verbindungen der Formel Ia

$R^N$-L′-N-$B^2$-$R^C$ (Ia)

steht, in welcher

$R^N$ für einen Rest der Formel IIa steht;

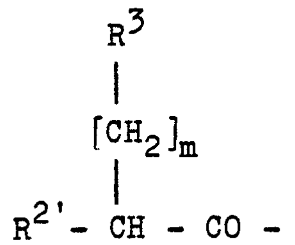

(IIa)

$R^{2'}$ für Wasserstoff oder einen Rest der Formel R″-$[A']_{n'}$-NH- steht;

$R^3$ Amino, Guanidino, ($C_1$-$C_3$)-Alkylamino oder Di-($C_1$-$C_3$)-alkylamino bedeutet;

m eine ganze Zahl von 1-6 bedeutet;

A′ für einen Rest der Formel -NH-$CR^4R^5$-CO- steht;

R″ Wasserstoff, ($C_1$-$C_6$)-Alkanoyl, ($C_7$-$C_{11}$)-Aroyl, das im aromatischen Teil gegebenenfalls durch ($C_1$-$C_4$)-Alkyl, ($C_1$-$C_4$)-Alkoxy, ($C_1$-$C_4$)-Alkylthio, Halogen, Carbamoyl, ($C_1$-$C_4$)-Alkoxycarbonyl und/oder Sulfamoyl mono- oder disubstituiert oder durch Methylendioxy monosubstituiert ist, ($C_5$-$C_7$)-Cycloalkyl-($C_1$-$C_3$)-alkanoyl oder ($C_6$-$C_{14}$)-Aryl-($C_1$-$C_3$)-alkanoyl bedeutet, wobei in den Resten R″ ≠ Wasserstoff eine -$CH_2$-Gruppe gegebenenfalls durch -O- oder -S- ersetzt ist;

n′ 0 oder 1 ist;

$R^4$ und $R^5$ gleich oder verschieden sind und Wasserstoff, ($C_1$-$C_6$)-Alkyl oder ($C_7$-$C_{11}$)-Aralkanoyl bedeuten;

L′ für den Rest einer lipophilen, neutralen α-Aminosäure, vorzugsweise für Pro, D-Pro oder einen Rest der Formel -NH-CH($R^6$)-CO- steht;

$R^6$ ($C_1$-$C_6$)-Alkyl, das gegebenenfalls durch Hydroxy, ($C_1$-$C_6$)-Alkoxy, ($C_1$-$C_6$)-Alkoxycarbonyl, Carbamoyl oder R″-NH monosubstituiert ist, wobei R″ wie oben definiert ist, aber nicht Wasserstoff sein kann, oder ($C_7$-$C_{11}$)-Aralkyl, das am Aromaten gegebenenfalls durch ($C_1$-$C_6$)-Alkoxy monosubstituiert ist, oder Indol-3-ylmethyl bedeutet;

N für den Rest einer neutralen α-Aminosäure, vorzugsweise für Pro, D-Pro oder einen Rest der Formel -NH-CH($R^7$)-CO- steht;

$R^7$ ($C_1$-$C_6$)-Alkyl, das gegebenenfalls durch Hydroxy, ($C_1$-$C_6$)-Alkoxy, ($C_1$-$C_6$)-Alkoxycarbonyl, Carbamoyl oder R″-NH monosubstituiert ist, wobei R″ wie oben definiert ist, aber nicht Wasserstoff sein kann, oder ($C_7$-$C_{11}$)-Aralkyl, das am Aromaten gegebenenfalls durch ($C_1$-$C_6$)-Alkoxy monosubstituiert ist, oder Indol-3-ylmethyl bedeutet;

$B^2$ für den Rest einer basischen α-Aminosäure, vorzugsweise für einen Rest der Formel IIIa

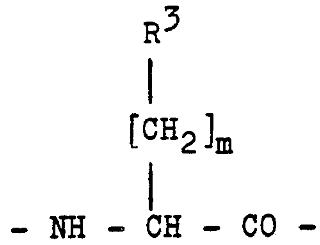

(IIIa)

worin $R^3$ und m wie oben definiert sind, steht;

$R^C$ für eine Rest der Formel $-NR^9-CH(R^8)-(CO)_p-R^1$ steht;

$R^8$ eine lipophile Seitenkette bedeutet, die vorzugsweise wie $R^7$ definiert ist, wobei vorhandene CH-, $CH_2$- oder $CH_3$-Reste in $\beta$-Stellung bezüglich -NH- gegebenenfalls monohydroxyliert sind, und

$R^9$ Wasserstoff bedeutet;

oder

$R^8$ und $R^9$ zusammen $-[CH_2]_3-$ oder $-[CH_2]_4-$ bedeuten;

p , 0 oder 1 ist;

$R^1$, im Falle von p = 0 für Wasserstoff, Hydroxy oder $(C_1-C_6)$-Alkoxy steht;

$R^1$ im Falle von p = 1 für $OR^{10}$ oder $NR^{10}R^{11}$ steht;

und

$R^{10}$ und $R^{11}$ gleich oder verschieden sind und Wasserstoff, $(C_1-C_6)$-Alkyl oder $(C_7-C_{11})$-Aralkyl bedeuten; oder $NR^{10}R^{11}$ für Pyrrolidino, Piperidino oder Morpholino steht;

oder

p = 1 ,ist,

$R''$ und $R^1$ gemeinsam eine Bindung bedeuten und die übrigen Reste wie oben definiert sind,

sowie deren physiologisch verträgliche Salze, verwendet wird.

3. Verwendung gemäß Anspruch 1 oder 2 zur Herstellung einer Zubereitung gemäß Anspruch 1, dadurch gekennzeichnet, daß Verbindungen der Formel I angewendet werden, in der

a) X Wasserstoff, $(C_1-C_5)$Alkoxycarbonyl, $(C_6-C_{12})$-Aryloxycarbonyl, $(C_7-C_{13})$-Aralkyloxycarbonyl, $(C_1-C_6)$-Alkanoyl, $(C_7-C_{13})$-Aroyl, Arginyl, Lysyl oder Arginyl-Arginyl bedeutet, X fehlen kann, gegebenenfalls in $\alpha$-Stellung verzweigtes und gegebenenfalls in $\omega$-Stellung durch Amino oder Guanidino monosubstituiertes $(C_1-C_{12})$-Alkylcarbonyl oder $(C_3-C_8)$-Cycloalkylcarbonyl bedeuten oder für Ser, Thr oder Q, jeweils in ihrer L- bzw. D-Konfiguration oder Ser-Ser, Thr-Thr, Ser-Thr, Pro-Ser, Pro-Thr, Thr-Pro oder Ser-Pro, vorzugsweise Ser-Ser steht, wobei jede Aminosäure in ihrer L- bzw. D-Konfiguration vorliegen kann und die N-terminale Aminogruppe der Aminosäure oder des Dipeptid-Restes frei vorliegt oder durch $(C_1-C_5)$-Alkoxycarbonyl, $(C_6-C_{12})$-Aryloxycarbonyl, $(C_7-C_{13})$-Aralkyloxycarbonyl, $(C_1-C_6)$-Alkanoyl, $(C_7-C_{13})$-Aroyl, Arginyl, Lysyl, $\epsilon$-Aminocapropyl oder Arginyl-Arginyl acyliert ist.

V Cys oder, falls X fehlt, Mercaptopropionsäure, Mercaptoessigsäure oder Mercaptobuttersäure bedeutet;

A Phe, Tyr(Me), Tyr($Bu^t$), 4-Halogenphenylalanin oder einen L-2-Thienylalanin-Rest, vorzugsweise Phe bedeutet;

$A^1$ Gly, Ala oder D-Ala bedeutet;

$A^2$ Gly, Ala oder D-Ala bedeutet; oder

$A^1$ und $A^2$ zusammen gegebenenfalls verzweigtes $\omega$-Amino-$(C_3-C_8)$-alkylcarbonyl bedeutet;

B Arg oder Lys bedeutet;

C Ile, Phe, Leu, Val, Cyclohexylalanin-, tert.-Butylglycin-, Neopentylglycin- oder einen L-2-Thienylalanin-Rest bedeutet;

D Asp, Glu, Gln, Asn, Leu, Ile, Trp, Asp($OBu^t$), Glu($OBu^t$), Glu(OBzl) oder einen 2-Thienylalanin-Rest, vorzugsweise Asp, Glu oder Leu bedeutet, wobei die Aminosäuren jeweils in ihrer L- oder D-Konfiguration vorliegen können;

E Gln, Thr oder Pro bedeutet;

F Ser, Thr oder Ala, vorzugsweise Ser oder Ala, jeweils in ihrer L- oder D-Konfiguration bedeutet;

G Gly, Ala oder D-Ala bedeutet

und

Z für ein Rest der Formel II steht, worin

H Asn, Ser, Pro oder Thr, vorzugsweise Pro, Thr oder Asn, jeweils in ihrer L- oder D-Form, oder Gly bedeutet;

I Ser, Thr, Ala oder Ser($Bu^t$), vorzugsweise Ser, jeweils in ihrer L- oder D-Form bedeutet;

J Phe, Tyr(Me), Cyclohexylalanyl oder einen L-2-Thienylalanin-Rest, vorzugsweise Phe bedeutet;
K Arg, Lys, Orn oder eine Bindung bedeutet;
L Tyr, Q, Gly, Tyr(Me) oder eine Bindung bedeutet;
M Arg-OH, Arg-$NH_2$, OH, OR, $NH_2$, NHR′, Gly-Lys-Arg-OH oder Gly-Lys-Arg-$NH_2$ bedeutet;
R unverzweigtes ($C_1$-$C_6$)-Alkyl bedeutet und
R′ -$[CH_2]_n$-$NH_2$ oder -$[CH_2]_n$-NH-C(NH)$NH_2$, wobei n ganzzahlig ist und für 3-8 steht, bedeutet, oder

b) X- $\overset{3}{V}$ -A-A[1]-A[2]- und -Gly-Ala-E-F-G-Leu-Gly- $\overset{19}{C}$ y s s-Z fehlen und der Rest -B-C-D-B-C- für Verbindungen der Formel Ia steht, in welcher
L′ für den Rest von Isoleucin, Valin, Threonin, Serin, O-($C_1$-$C_9$)-Alkylthreonin, O-($C_1$-$C_9$)-Alkylserin, Leucin, Prolin oder des ω-($C_1$-$C_6$)-Alkyl-vorzugsweise tert. Butylesters von Glutaminsäure oder Asparaginsäure steht;
N für den Rest von Valin, Isoleucin, Leucin, Phenylalanin, Tryptophan, gegebenenfalls O-($C_1$-$C_6$)-alkyliertem Tyrosin, Glutamin, Asparagin, Glutaminsäure-γ-($C_1$-$C_6$)-alkylester oder Asparaginsäure-β-($C_1$-$C_6$)-alkylester oder ε-Acyl-lysin steht,
und
$B^2$ Arg, D-Arg, Lys oder D-Lys bedeutet, oder deren physiologisch verträgliche Salze.

4. Verwendung gemäß einem der Ansprüche 1 bis 3 zur Herstellung einer Zubereitung gemäß Anspruch 1, dadurch gekennzeichnet, daß eine Verbindung der Formel I angewendet wird, in der
X Wasserstoff, Arginyl oder Arginyl-Arginyl, Ser, Q oder Ser-Ser bedeutet, wobei Ser und Q jeweils in der L- oder D-Konfiguration vorliegen können und die N-terminale Aminogruppe des Aminosäure- oder Dipeptid-Restes frei vorliegt oder durch Arginyl oder Arginyl-Arginyl acyliert ist oder X fehlen kann;
V Cys oder, falls X fehlt, Mercaptopropionsäure bedeutet;
A Phe bedeutet;
B Arg oder Lys bedeutet,
C Ile, Leu, Val oder einen L-2-Thienylalanin-Rest bedeutet;
D Asp, Gln, Leu, Ile, Asp($OBu^t$), Glu($OBu^t$), Tyr($Bu^t$) oder Tyr(Me) bedeutet;
E Gln, Thr oder Pro bedeutet;
F Ser oder Ala jeweils in ihrer L- oder D-Konfiguration bedeutet;
G Gly, Ala, oder D-Ala bedeutet
und
Z für einen Rest der Formel II steht worin
H Asn, Pro oder Thr, jeweils in ihrer L- oder D-Form, oder Gly bedeutet;
I Ser, Thr oder Ala jeweils in ihrer L- oder D-Form bedeutet;
J Phe, Tyr(Me), Cyclohexylalanyl oder einen 2-Thienylalanin-Rest bedeutet;
K Arg, Lys oder eine Bindung bedeutet;
L Tyr, Tyr($Bu^t$) oder eine Bindung bedeutet;
M OH, $NH_2$, NHR′, Gly-Lys-Arg-OH oder Gly-Lys-Arg-$NH_2$ bedeutet
und
R′ -$[CH_2]_2$-$NH_2$ oder -$[CH_2]_n$-NH-C(NH)$NH_2$ bedeutet, wobei n geradzahlig ist und für 3-8 steht.

5. Verwendung gemäß einem der Ansprüche 1 bis 4 zur Herstellung einer Zubereitung gemäß Anspruch 1, dadurch gekennzeichnet, daß ANF, dessen Teilsequenz oder Analogon für die entsprechenden Anwendungsformen mit pharmazeutisch geeigneten Trägerstoffen bzw. Hilfsstoffen kombiniert wird.

6. Verfahren zur Herstellung einer Zubereitung gemäß einem der Ansprüche 1 bis 5, dadurch gekennzeichnet, daß man den Wirkstoff mit den geeigneten Träger-, Hilfs- und/oder Zusatzstoffen in eine geeignete Darreichungsform bringt.